# EUROPEAN PATENT APPLICATION

(11) **EP 3 679 859 A1**
(43) Date of publication of application: **15.07.2020**
(21) Application number: 19000596.7
(22) Date of filing: 27.12.2019
(51) Int. Cl.: A61B 5/024, A61B 5/16, A61B 5/00, A61B 5/18

(54) **A PERIPHERAL DEVICE FOR SUPPORTING PSYCHOLOGICAL EXAMINATIONS**

(30) Priority: 08.01.2019 PL 42851919
(71) Applicant: Politechnika Slaska, 44-100 Gliwice (PL)
(72) Inventor: Dobrowolska, Malgorzata, 40-540 Katowice (PL); Socha, Vladimir, 04022 Kosice (SK); Bereska, Damian, 44-100 Gliwice (PL); Jedrasiak, Karol, 44-190 Mikolow (PL); Nawrat, Aleksander, 41-807 Zabrze (PL); Kwiatkowski, Jan, 41-933 Bytom (PL)

(57) **Abstract**

A peripheral device for supporting psychological examinations, characterised in that an element to monitor a response at conducting a psychological test comprises at least 10 transparent buttons (2), LED illumination (3), is provided with a piezoelectric sensor (4) and a sensor to register cardiac activity (11), being together indicators of psycho-physiological state. The control of illumination of a defined number of buttons (2) by means of the LED diodes (3) is a sliding multi-position switch (4) connected with a microprocessor unit (6). Particular piezoelectric elements (5) are active only when particular LED diodes (3) are illuminated. The data are stored in an internal memory (8) of the device with the possibility to send the data to the computer wireless. The device uses a Bluetooth interface (7) to communicate with the own periferal devices.

## Description

The object of the invention is a peripheral device for supporting psychological examinations in terms of analysing a level of the participant's decisiveness in response giving. The scope of the device application is based on general psychology and specialist psychology.

Over many years, psychological studies were performed by way of manually handled psychological tests conducted in the presence of a psychologist whose task was to further analyse and interpret the obtained information. Technology facilitates the process of performance of psychological tests due to the possibility to support the analysis of the test results with hardware and software solutions. Psychological tests may be installed on a computer and the participant fills in thereof with the use of a keyboard.

In Poland, assessment in case of so called high risk occupations is compulsory. It is conducted by every psycho-technical office (entities entitled thereto). An example of high risk occupation in industry are the jobs involving work at chemical installations for production of toxic gases or gases forming explosive mixtures with air; work at open radioactive sources of class I and II; drill operator work and basic machinery operator work in opencast mining; work related to site clearing of explosive objects in underground mining facilities; work related to operation and movement maintenance of nuclear reactors, accelerators, generators, neutrons, chambers for production of radioactive sources, etc..

Having the above-mentioned examples in mind, every specialist, including occupation medicine and organization specialists, individually prepare packets, available on the market psychological tests such as personality tests, organic tests, intelligence test or psycho-social competency tests.

Assessment packets to verify psycho-physical and psycho-social potential of high risk groups may be administered by: every assessment office, psycho-technical (within the compulsory assessment n the occupational medicine); every job agency (within the recruitment commissioned by an industrial entity); management divisions of industrial companies (employing people of so called high risk occupations). Within the scope of the conducted psychological assessment specific for a job a significant factor that may indirectly influence the test results is the measurement of a level of the participant's decisiveness. Analysis and classification of the measurement data obtained by way of the conducted tests constitute an important element of the art of psychology.

From the patent description US20110151418A1 it is known a device to obtain, collect and analyse psychological data and/or psycho-pathological data in order to select the relevant treatment method for the identified condition. The primary feature of the patent is a wearable, telemetric feature of the device allowing to monitor and assess certain particular questionnaires right away. It means that the participant may be assessed in specified daily schedules and in different psycho-physiological states. The device has been designed as a watch of a particular type of operation.

From the patent description US3438141A it is known a device to support psychological assessment with the task thereof to present the questionnaire questions as well as to share the data input method and to collect the data obtained when testing. The above mentioned invention relates to a psychological test, and at the same time to an assessment device, conducted with the use of a dice. This device also responds to the need to provide psychological tests that are not based on questionnaires.

Both above mentioned examples use the tests and test concepts focused on particular cases. Nowadays, despite the attempt to digitalise standard psychological tests (paper versions) they are still present in assessments. It should be mentioned that a questionnaire form for tests is still present and requires to be analysed by a psychologist. The tests that usually consist of plurality of questions are time consuming and they do not provide enough information on psycho-physiological state of the monitored subject. Another limitation is inability for those concepts to create easily acceptable aggregated population statistics.

The limitations may be removed due to the present technical solution that is an interface between the described form of testing with simultaneous monitoring of complementary psycho-physiological indicators. The object of the invention is to increase the efficiency of psychological testing as well as to provide detailed and objective data that additionally allow to determine the decisiveness ability of the tested subject.

The object of the invention is to support all psychological tests that will be created in an academic and experimental versions that are available on the market but do not exist in the computer version, and for the individual use by researchers in research and psychological laboratories that develop their own research and create original process of measurement of psychological variables.

The above mentioned limitations are to the large extent removed by the present peripheral device supporting psychological examinations. The subject of the invention is based on standard psychological tests filled out with a "pencil". Each of the respondents ticks particular answer fields. That is why in the device there has been ergonomically designed the ten of buttons arranged such that they would fit both digits of left and right hand. Particular buttons are illuminated (being illuminated) to indicate they are activated. The button activation depends on the question. In the case of the questions where the answer is a value in the scale from 1 to 10 (for example 1-never, 2-almost never, seldom, 3-seldom, 4-sometimes, 5-from time to time, 6-usually, 7-often, 8-very often, 9-almost always, 10-always). Those all 10 buttons that are designed in one colour, their scale is from left to right. If the requested answer is in the scale 1-4 (for example 1-not, 2-rather not, 3-yes soon, 4-rather yes) only 4 buttons are illuminated. Such a rule is applied for all possible options of the answers.

The number of the active buttons is set prior to actual measurement with the use of a rotary disc mounted in the device. The setting takes place prior to the very measurement and is based on the standards for psychological tests where the answers for the same type of questionnaire are in the same scale. It enables testing offline when digital psychological tests are not available.

Under each button there is a piezoelectric sensor detecting force of the impact. Moreover, the device is typically connected with a wireless ECG module that sends information on RR intervals (pauses between heart beat-to-beat). The signal, together with the impact force value, may be used in a post-hoc assessment of the psycho-physiological state of the participant. All the data is stored mainly in the internal memory of the device, and when the device is paired with relevant software then the data is sent to the personal computer via a Bluetooth interface.

### Summary description of the figures (the device is presented in the preferred embodiment)

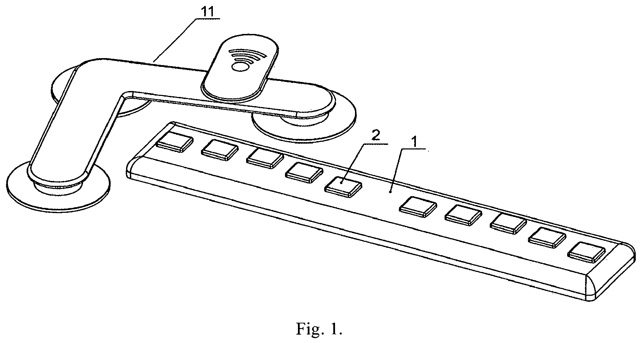

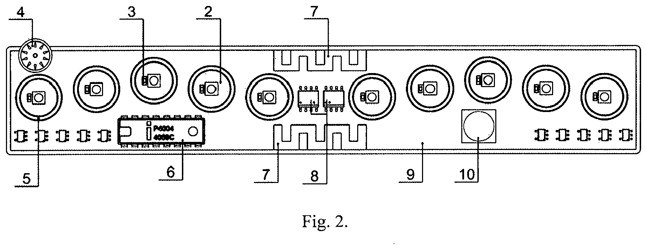

Fig. 1 is an isometric view of the device. Fig. 2 is a view of the device from the top under the cover.

### Detailed description of the drawings

The device for visualisation from Fig. 1 comprises a plastic slot 1 of the device forming a cover of the device. The output elements are matt transparent buttons (10x) 2 illuminated by diodes 3. The setting of the number of the illuminated buttons by means of the diodes is with the use of a wheel 4. The buttons 2 are located on the piezoelectric sensors 5 detecting the impact intensity. The sliding multi-position switch 4, together with the sensors 5 and the diodes 2, is mounted on a printed circuit board 9 connecting the elements thereof with a microprocessor unit 6. The microprocessor unit 6 controls the transfer logic between the superior device and inferior device (slave) by the Bluetooth module via integrated aerials 7, as well as controls the collection of data which subsequently stores in an internal memory 8. The aerials 7 provide communication with the peripheral device in order to collect signals of cardiac activity 11 by sending data to the computer. The device is powered by a replaceable battery 10.

## Claims

1. A peripheral device for supporting psychological examinations, **characterised in that** an element to monitor a response at conducting a psychological test comprises at least 10 transparent buttons (2), LED illumination (3), is provided with a piezoelectric sensor (4) and a sensor to register cardiac activity (11), being together indicators of psycho-physiological state.

2. The device according to claim 1, **characterised in that** the control of illumination of a defined number of buttons (2) by means of the LED diodes (3) is a sliding multi-position switch (4) connected with a microprocessor unit (6).

3. The device according to claims 1 and 2, **characterised in that** particular piezoelectric elements (5) are active only when particular LED diodes (3) are illuminated.

4. The device according to any of the preceding claims, **characterised in that** the data are stored in an internal memory (8) of the device with the possibility to send the data to the computer wireless.

5. The device according to any of the preceding claims, **characterised in that** a source of the energy is a replaceable battery.

6. The device according to any of the preceding claims, **characterised in that** uses additional data collections registered by means of a wireless sensor for registration of cardiac electric activity (11).

7. The device according to any of the preceding claims, **characterised in that** uses a Bluetooth interface (7) to communicate with the own periferal devices.
